Europäisches Patentamt

(19) European Patent Office

Office européen des brevets

(11) EP 0 662 847 B1

## (12) EUROPEAN PATENT SPECIFICATION

(45) Date of publication and mention
of the grant of the patent:
**14.08.1996 Bulletin 1996/33**

(21) Application number: **93922334.3**

(22) Date of filing: **23.09.1993**

(51) Int. Cl.⁶: **A61L 15/60**, A61F 13/15,
C08J 5/18, B29C 35/06,
B32B 31/12

(86) International application number:
**PCT/US93/09022**

(87) International publication number:
**WO 94/07547 (14.04.1994 Gazette 1994/09)**

(54) **METHOD AND APPARATUS FOR MAKING COHESIVE SHEETS FROM PARTICULATE ABSORBENT POLYMERIC COMPOSITIONS**

VERFAHREN UND VORRICHTUNG ZUR HERSTELLUNG ZUSAMMENHÄNGENDER BAHNEN AUS ZUSAMMENSETZUNGEN VON ABSORBIERENDEN POLYMERTEILCHEN

PROCEDE ET DISPOSITIF DE FABRICATION DE FEUILLES COHESIVES A PARTIR DE COMPOSITIONS PARTICULAIRES POLYMERES ABSORBANTES

(84) Designated Contracting States:
**AT BE CH DE DK ES FR GB GR IE IT LI LU NL PT SE**

(30) Priority: **02.10.1992 US 955638**

(43) Date of publication of application:
**19.07.1995 Bulletin 1995/29**

(73) Proprietor: **THE PROCTER & GAMBLE COMPANY Cincinnati, Ohio 45202 (US)**

(72) Inventors:
• **REZAI, Ebrahim**
  **45239, Ashiya-shi, Hyogo 659 (JP)**
• **LAHRMAN, Frank, Henry**
  **Cincinnati, OH 45241 (US)**

• **IWASAKI, Toshiaki**
  **Machida, Tokyo 194 (JP)**
• **BENSON, Douglas, Herrin**
  **West Harrison, IN 47060 (US)**
• **KOLODESH, Michael Sulya**
  **Higashinada-ku, Kobe 658 (JP)**

(74) Representative: **Bottema, Johan Jan et al**
  **Procter & Gamble GmbH**
  **Patent Department**
  **Sulzbacher Strasse 40-50**
  **65824 Schwalbach am Taunus (DE)**

(56) References cited:
**EP-A- 0 493 011**         **US-A- 4 310 593**
**US-A- 4 851 069**         **US-A- 5 102 597**

EP 0 662 847 B1

**Description**

FIELD OF THE INVENTION

The present invention relates to particulate absorbent polymeric compositions which, upon contacting liquids such as water or body exudates, swell and imbibe such liquids. More specifically, the present invention relates to an apparatus and method for making cohesive sheets from such compositions.

BACKGROUND OF THE INVENTION

"Particulate absorbent polymeric compositions" (PAPC) as used herein refers to particulate, substantially water-insoluble, absorbent hydrogel-forming, polymer materials which are capable of absorbing large quantities of liquids, such as water and body exudates, and which are further capable of retaining such absorbed liquids under moderate pressures. The absorption characteristics of such polymeric compositions make them especially useful for incorporation into absorbent articles such as diapers. For example, U.S. Patent No. 3,669,103 issued to Harper et al. on June 13, 1972 and U.S. Patent No. 3,670,731 issued to Harmon on June 20, 1972, both disclose the use of particulate absorbent polymeric compositions, also referred to as hydrogels, superabsorbent or hydrocolloid materials and referred to herein as particulate absorbent polymeric compositions (PAPC). In the past PAPC particles have been combined with conventional absorbent fibers, for example by incorporating PAPC's into tissue laminate materials for the production of diaper cores and the like.

However, combining PAPC with conventional absorbent fibers has posed many problems. One problem has been that absorbent cores of diapers or catamenials and the like made with this combination tend to be undesirably thick as the incorporation of PAPC into the core does not eliminate substantial amounts of fiber. Furthermore, the particles are not immobilized and are free to migrate during processing and/or use. Migration of particles can lead to material handling losses during manufacture as well as non-homogeneous incorporation of the particles into the structures in which the particles are being used.

Therefore, there has been a desire to make absorbent sheets or macro-structures from PAPC without the need to encapsulate the PAPC particles between tissue layers. The ability to make sheets of PAPC would facilitate the making of high capacity ultra-thin absorbent cores for diapers and the like, because it would substantially reduce or eliminate fibers in the absorbent core. Moreover, there would be no problem of particle migration if the PAPC were in sheet form. Another advantage is that PAPC can retain absorbed liquid under moderate pressure providing for good leakage protection and skin dryness properties. However, making sheets of particulate polymeric compositions for use in absorbent cores of diapers, catamenials, bandages and the like, has proven to be difficult. As the PAPC's absorb fluid they swell and become a gelatinous mass. If the particles are close to one another, as they would be in a sheet, they can coalesce, forming a gel barrier which blocks the flow of fluid. This phenomena is commonly referred to as gel blocking. The problem is that when the absorbing gelling particles are placed closely together there is a high resistance to liquid flow through the material due to the lack of stable interparticle capillary or liquid transport channels. This phenomenon is also a problem when the particles of PAPC migrate close together, as they would when placed between fiber layers as described above.

A method of overcoming gel blocking to make absorbent sheets from particulate absorbent polymeric compositions is disclosed in commonly assigned U.S. Patent 5,102,597 issued to Roe et al. on April 7, 1992. Roe et al. teaches that by initially mixing the PAPC particles with a solution of a non-ionic cross-linking agent, water and an organic solvent, one can substantially eliminate gel blocking in PAPC macro-structures. Roe et al. teaches the making of absorbent sheets of PAPC by mixing the PAPC particles, the cross-linking agent, water and the organic solvent into the hopper of a mixer to form agglomerated PAPC particles. The mixture of agglomerated PAPC particles are then fed into a compactor and thereafter passed through compression rolls to form sheets. The sheets are then cured in an oven and plasticized with water and glycerol to make them flexible.

However, the above described method for making PAPC sheets has been found to be impractical. The handling and metering of the sticky, wet agglomerated particulate absorbent polymeric compositions was difficult due to the stickiness of the mixture. The sheets tended to lack uniformity and be non-continuous. Furthermore, it was very difficult to control the density, thickness and width of the sheets due to the poor handling characteristics of the wet agglomerated PAPC.

It is therefore an object of the present invention to provide a method and apparatus for making cohesive sheets from particulate absorbent polymeric compositions which overcomes the problems associated with handling wet and sticky agglomerated PAPC's.

It is another object of the present invention to provide a method and apparatus for making cohesive sheets from PAPC wherein the PAPC is layered in its dry state, before adding water and/or cross-linking agents, so that handling and metering the particles is easier.

It is another object of the present invention to provide such a method and apparatus that will produce a cohesive sheet of PAPC having a substantially uniform density and thickness.

It is yet another object of the present invention to provide a method and apparatus for making cohesive sheets from PAPC which is inexpensive and easy to implement.

The aforementioned and other objects of the invention will become more apparent hereinafter.

## SUMMARY OF THE INVENTION

In accordance with an aspect of the present invention there is provided a method for making a cohesive sheet from particulate substantially water insoluble, absorbent hydrogel-forming polymer materials. The method comprises the steps of continuously layering a predetermined amount of dry absorbent gelling particles so as to substantially cover a predetermined area of a support means. The layered particulate material in the predetermined area of the support means is then sprayed with an amount of liquid mixture comprising water and a cross-linking agent for the polymers in the particulate material. The amount sprayed is sufficient to cause effective surface cross-linking of the particulate material when cured. Pressure is then applied to the particulate material by passing the layer of particulate material through a pair of opposing pressure applicators thereby, forming a sheet of particulate absorbent polymeric compositions.

In accordance with another aspect of the present invention the above described method further includes the step of curing the cohesive sheet by allowing it to sit at a predetermined temperature for a predetermined amount of time in order to provide for effective surface cross-linking of the particulate material. The cured cohesive sheet will then absorb liquid upon contact and remain in sheet form without separating into individual particles. Furthermore, the layering and spraying steps described above can be repeated a number of times.

In accordance with yet another aspect of the present invention there is provided an apparatus for making a cohesive sheet from particulate substantially water insoluble, absorbent hydrogel-forming polymer materials. The apparatus comprises a support means, a means for continuously layering a predetermined amount of dry absorbent gelling particles onto the support means so as to substantially cover a predetermined area of the support means, and a means for spraying the layered particulate material in the predetermined area with an amount of a liquid mixture comprising water and a cross-linking agent for the polymers in the particulate material, the amount sprayed being sufficient to cause effective surface cross-linking of the layered particulate material after curing. The apparatus further comprises a pair of opposing pressure applicators for applying pressure to the layered particulate material by passing the material therethrough, thereby forming a cohesive sheet.

## BRIEF DESCRIPTION OF THE DRAWINGS

While the specification concludes with claims particularly pointing out and distinctly claiming the subject invention it is believed that the same will be better understood from the following description taken in conjunction with the accompanying drawings in which:

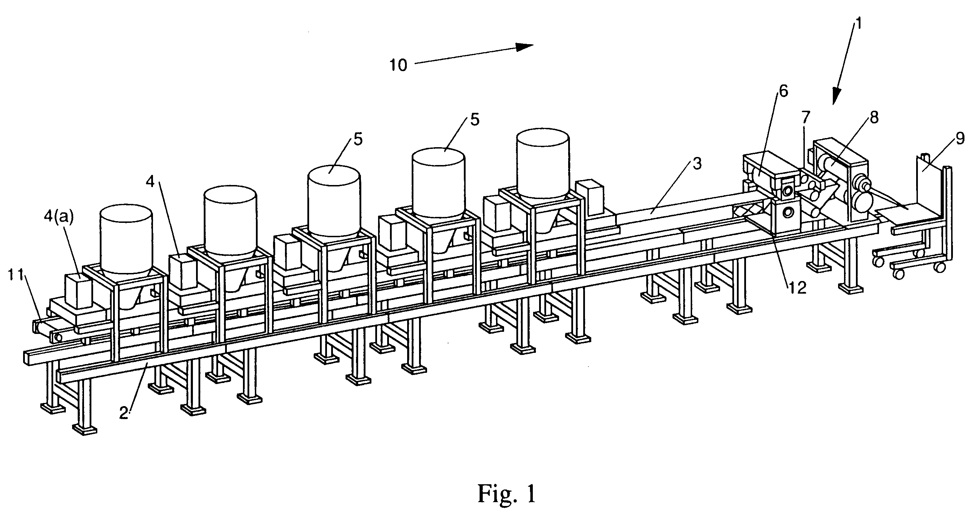

Figure 1 is a simplified perspective view of an apparatus for making an absorbent sheet from particulate absorbent polymeric compositions using the method of the present invention.

## DETAILED DESCRIPTION OF THE INVENTION

Referring now to the drawings in detail wherein like numerals indicate the same element throughout the views there is shown in Figure 1 a simplified perspective view of an apparatus 1 for making cohesive sheets from particulate absorbent polymeric compositions. Apparatus 1 has frame 2 for supporting its various components. The Apparatus comprises a support means, shown in the drawings as moving conveyor 3 which moves in the direction of arrow 10. The conveyor first passes under an initial sprayer 4a. After passing under the initial sprayer 4a, the conveyor passes under at least one means for continuously layering a predetermined amount of PAPC onto the conveyor. This is shown in the figure as PAPC feeder 5. The conveyor also passes under at least one means for spraying an amount of liquid mixture onto the layered PAPC on the conveyor. This is shown in the figure as sprayer 4. The apparatus further comprises a pair of non-planar opposing pressure applicators down stream from the feeders 5 and sprayers 4. The pressure applicators are shown in the figure as a pair of compaction rolls 6. Also shown in Figure 1 as being part of apparatus 1 is a slitting and transfer conveyor 7, knife and anvil rolls 8, and a sheet accumulator 9. The method for making absorbent sheets from PAPC's using the above apparatus is easily understood from the description given below.

A support means is provided for layering the PAPC. It is preferred that the surface be somewhat rough so that it has good release properties. The preferred embodiment for the support means for the present invention is a movable conveyor, shown in the Figure as conveyor 3. Conveyor 3 can be a flat belt conveyor that has good release properties, such as polyurethane, which is commonly used in the food industry. The width of the conveyor is determined by the desired sheet size. The conveyor generally moves in the direction of arrow 10 from point 11, were the initial sprayer 4a is located, to a point 12, where the knife and anvil rolls 8 are located. Conveyor 3 would typically be an endless conveyor as shown in the Figure.

In a preferred embodiment conveyor 3 first passes under an initial sprayer 4a, where the conveyor is sprayed with a an amount of a liquid mixture so as to cover a predetermined area of the conveyor. The composition and amount of liquid mixture used depends on the type of PAPC sheet being made and is discussed in further detail below. In a preferred embodiment, the liquid mixture for all sprayers is the same and contains a solution comprising water and a cross-linking agent for the polymer materials in the PAPC. The liquid mixture may also contain other compositions such as plasticizers, to make the resultant sheet more flexible and organic solvents. The initial spraying insures that the bottom part of the first applied PAPC layer is exposed to the liquid mixture. Furthermore, the wet conveyor surface will prevent the subsequently applied PAPC particles from bouncing away from their desired placement. However the initial spraying step is not absolutely necessary especially when the first layer of PAPC particles to be placed on the conveyor is thin. Moreover, the problem of particle bouncing is not as pronounced when the conveyor travels at slower speeds.

The conveyor then passes under a feeder 5 where a predetermined amount of dry PAPC is layered onto the predetermined area of the conveyor. The amount of PAPC to be layered onto the conveyor depends on a number of factors including, but not limited to: the desired density of the resultant absorbent sheet, the number of PAPC layering steps to be performed, the size of the PAPC particles being used and the desired width of the resultant PAPC sheet. At a minimum the predetermined amount should be enough to substantially cover a predetermined area of the conveyor with a layer of PAPC one particle in thickness. However, as will be described below, the density of the resultant PAPC sheet has preferred upper and lower limits. Therefore, for a resultant sheet having a given density one can begin to determine how the predetermined amount changes for given conditions. First, the more layering steps that are to be performed, the less the predetermined amount would have to be for each layer. Second, the smaller the size of the particles, the less the predetermined amount will be. This is because when using smaller particle sizes each layer will have a greater number of particles stacked on top of one another. The greater the number of particles stacked on top of one another per layer, the less likely the liquid mixture will penetrate the entire layer and treat each particle. Therefore, the smaller the particles the lesser the amount of PAPC for each layer.

Any suitable feeding or metering device for dry powders can be used. The feeder must be capable of distributing the PAPC particles in a thin and preferably wide layer. Thinner layers of PAPC applied to the conveyor ensure that all of the particles are treated during subsequent spraying steps and wider layers will increase production output. Furthermore, to have a more uniform distribution of PAPC on the support means it is desired that the relative humidity of the surrounding atmosphere be less than or equal to 50%. Vibrating feeders have been shown to be adequate for layering the dry particulate PAPC onto the conveyor. An example of a suitable vibrating feeder is a Super Feeder model #2106E-003S4 commercially available from Solids Flow Control P.O. Box 410767, 14201-A South Lakes Drive Charlotte N.C. 28241-0767. This feeder has a weight feed-back control system for accuracy.

The conveyor 3 then passes under a sprayer 4 where the predetermined area of the conveyor having the PAPC thereon is sprayed an amount of liquid mixture comprising water and a cross-linking agent for the PAPC. In a preferred embodiment the liquid mixture is the same as that used in initial sprayer 4a. The amount of liquid mixture sprayed is sufficient to cause effective surface cross-linking of the PAPC particles after curing (curing is discussed in detail below). In general the amount of liquid mixture (LM) is related to the amount of PAPC that was layered above. The greater the amount of PAPC layered, the more liquid mixture that is needed to treat substantially all of the particles. A suitable liquid mixture taught by the hereinbefore incorporated Roe et al. reference for effective surface cross-linking of the PAPC is a solution of a non-ionic cross-linking agent, water and an organic solvent. Compositions of the liquid mixtures that can be used with various PAPC's is discussed in detail below.

Many commercially available sprayers may be suitable. The sprayer used must deliver a substantially uniform mist, atomized spray and should have a low impact force to avoid possible blow off of PAPC particles. One sprayer that has been found to work well is a model 6218-1/4 JAU atomized air actuated nozzle assembly available from Spraying Systems Co., Wheaton, IL. 60188.

The metering and spraying steps can then be repeated a number of times depending on the desired density of the absorbent PAPC sheet. If one were to layer a large amount of PAPC in one step, any subsequent spraying step would not be able to penetrate the entire thickness of the layer in order to treat the PAPC particles. That is why thin layers of PAPC are layered onto the conveyor and sprayed. Thereafter, the layering and spraying steps can be repeated until the amount of PAPC necessary to form a sheet of the desired density has been layered. The combined amount of liquid mixture sprayed is sufficient to cause effective surface cross-linking of the PAPC particles after curing. When the layered PAPC is sprayed with the liquid mixture, the PAPC will often loosely adhere together to form a web. This allows the layered PAPC to be fed through the nip of the compaction rolls 6.

When the metering and spraying steps are done a number of times and the initial spraying step is to be performed, as described above, the first layer of PAPC is exposed to two spraying applications. Therefore the initial spraying step and the first post-layering spraying step each need only spray half the amount of liquid mixture needed to treat that amount of PAPC layered onto the conveyor. That is the initial sprayer and the first post-layering sprayer will spray half as much as the other sprayers.

After all of the layering and spraying steps have been performed, the conveyor 3 then moves the layered PAPC and delivers it to a pair of opposing pressure applicators. The pressure applicators as shown in Figure 1 take the form of compaction rolls 6. However, as will be appreciated to those skilled in the art, an intermittent conveyor method could be used wherein the apparatus would employ opposing plates or platens to compress the web. When the layered PAPC is sprayed with the liquid mixture, the PAPC will often loosely adhere together to form a web. This allows the layered PAPC to be fed through the nip of the compaction rolls 6.

In a preferred embodiment the compaction rolls 6 are non-planar or have a rough surface. As the PAPC passes through the compaction rolls the pressure causes the sheet to expand. The rough surface of the rolls 6 reduces the sliding effect between the rolls and the PAPC in contact with the rolls. This in turn reduces expansion of the sheet in both the machine direction 10 and cross-machine direction. Machine direction expansion is undesirable because it requires the compaction rolls to speed up in order to match the machine direction expansion.

The compaction step of the process densifies the freely deposited layers of PAPC and sprayed solution into a sheet. Even if the sheet is cured, as described below, without the compaction, the PAPC would not hold together upon contact with the fluid it is intended to absorb and would not have the desired mechanical strength. The layered PAPC would most likely disperse into separate particles and the amount of absorption desired would not be obtained due to the migration and gel blocking problems that would occur.

In a preferred embodiment compaction rolls 6 are cylindrical stainless steel rolls that are coated with a plasma coating thereby giving the rolls a rough surface and causing them to release the sheet more easily after compaction. The plasma coating gives the rolls a rough surface and in addition and independently to that it provides the rolls with a surface having good release properties. Examples of suitable coatings include coating #'s 934 and 936 available from Plasma Coatings, Inc., Waterbury, Ct. 06702. The gap between the compaction rolls determines the amount of compaction applied to the PAPC.

The amount of compaction to be applied to the PAPC is one of the many factors that determine the density of the resultant sheet of PAPC. Other factors include the amount of PAPC and liquid mixture deposited onto the support means during the layering and spraying steps, the roughness of the rolls, the type of PAPC particles being used and many others. In order to insure the integrity of the PAPC sheet the minimum density for such a sheet is approximately 0.70 grams/cubic centimeters . However, in order to prevent gel blocking the maximum density for such a sheet is approximately 1.10 grams/cubic centimeters.

In one embodiment of the present invention the apparatus includes a slitter to trim the edges of the layered PAPC prior to compaction. This is because the edges of the layered PAPC have a less uniform density than the rest of the layer. The edges are subjected to inconsistent application of liquid mixture and PAPC due to the conveyor belt movement in the cross-machine direction. As will be appreciated to those skilled in the art the slitter can be a regular circular knife working against a hard surface such as a transfer conveyor belt.

After passing the web through compaction rolls 6, a sheet of PAPC is formed and collected in accumulator 10. Accumulator 10 can take the form of a wind-up roll that rolls up the PAPC sheet into a single roll of a desired size. When the desired size roll is obtained the apparatus has a second slitter to cut the PAPC sheet. As will be appreciated by those skilled in the art this second slitter can take the form of a knife and anvil roll.

In order to provide for effective surface cross-linking of the PAPC and to make the sheet of PAPC absorbent and to provide the necessary mechanical strength for the sheet, the additional step of curing the sheet could be performed. This is done so that the cross-linking agent reacts with the polymer material of the PAPC particles, while maintaining the physical association of the PAPC particles, to provide effective surface crosslinking in the PAPC particles in the aggregate sheet. The method for curing the sheets depends on the type of PAPC materials being used the composition of the liquid mixture. Curing usually involves subjecting the sheets to a predetermined temperature for a predetermined amount of time. The specific curing steps involved for the various cross-linking agents used is discussed below in conjunction with the specific chemical structure of the various liquid mixtures.

If a cationic amino-epichlorohydrin adduct is used as the cross-linking agent, the relatively reactive cationic functional groups of the adduct causes the crosslinking reaction with the polymer material of the PAPC particles to occur at relatively low temperatures. Indeed, this crosslinking reaction (curing) can occur at ambient room temperatures. Such ambient temperature curing is particularly desirable when the liquid mixture additionally contains a plasticizer, such as a mixture of water and glycerol. Curing at significantly above ambient temperatures can cause the plasticizer to be driven off due to its volatility, thus necessitating an additional step to plasticize the resulting interparticle bonded aggregate. Such ambient curing is typically carried out at a temperature of from about 18° to about 35°C for from about 12 to about 48 hours. Preferably, such ambient curing is carried out at a temperature of from about 18° to about 25°C for from about 24 to about 48 hours.

Although the crosslinking reaction between the cationic amino-epichlorohydrin adduct and the polymer material of the PAPC particles can occur at ambient temperatures, such curing can also be carried out at higher temperatures to speed up the reaction. Higher temperature curing typically involves heating the treated and associated PAPC particles to cause the crosslinking reaction between the adduct and the polymer material of the PAPC particles to occur in a shorter period of time, typically minutes. This heating step can be carried out using a number of conventional heating devices, including various ovens or dryers well known in the art.

Generally, heat curing can be carried out at a temperature above about 50°C for a period of time sufficient to complete the crosslinking reaction between the adduct and the polymer material of the PAPC particles. The particular temperatures and times used in heat curing will depend upon the particular cationic amino-epichlorohydrin adduct used and the polymer material present in the PAPC particles. If the cure temperature is too low, or the cure time too short, the reaction will not be sufficiently driven, resulting in sheets that have insufficient integrity and poor absorbency. If the cure temperature is too high, the absorbency of the PAPC particles can be degraded or the network crosslinks of these PAPC particles, depending upon the specific polymer materials used, can be degraded to such point that the resulting sheet is less useful for absorbing large quantities of liquids. In addition, if the cure time and temperatures are not appropriate, extractable levels of the resulting aggregates can be greater, thus increasing the incidence of that particular form of gel-blocking. Therefore, heat curing is generally carried out at a temperature in the range of from about 50° to about 205°C for from about 1 to about 20 minutes. Preferably, heat curing is carried out at a temperature of from about 180° to about 200°C for from about 5 to about 15 minutes. The actual time and temperatures used can vary depending upon the specific polymer materials used in making the PAPC particles, the specific adducts used, the thickness or diameter of the sheet involved, and like factors.

If a non-ionic cross-linking agent, such as glycerol, is to be used, as is described in said hereinbefore incorporated U.S. patent 5,102,597 issued to Roe et al., then the curing may be done by irradiation (e.g., ultraviolet, gamma- or X-radiation) or by a catalyst as an initiator and an activator, however, the crosslinking reaction is preferably thermally activated (heating). Heating activates and drives the reaction and drives off any volatiles present in the mixture. Such reaction conditions will generally involve heating the associated precursor particles and the nonionic crosslinking agent for certain times and at certain temperatures. The heating step can be carried out using a number of different apparatus as are known including the various ovens or driers as are known in the art.

Generally, the reaction is effected by heating to a sufficient temperature for a sufficient time to complete the crosslinking reaction. For each set of specific nonionic crosslinking agent(s) and polymer material of the precursor particles used, if the temperature is too low or the time is too short, the reaction will not be sufficiently driven resulting in fewer and weaker interparticle crosslink bonds thereby causing some loss of liquid permeability of the sheet upon swelling. If the temperature is too high, the absorbency of the precursor particles may be degraded or the network crosslinks of these precursor particles, depending upon the specific polymer materials, may be degraded to such a point that the resultant sheet is not useful for absorbing large quantities of liquids. In addition, if the time and temperatures are not correct, the extractable levels of the resultant aggregates may increase, thereby increasing the incidence of that form of gel blocking. Therefore, when nonionic crosslinking agents such as glycerol are used, the reaction will generally be carried out at a temperature in the range from about 120° C. to about 205° C., more preferably from about 180° C. to about 200° C. The time to complete the reaction, in the absence of catalysts, will generally be from about 15 minutes to about 2 hours, more preferably from about 30 minutes to about 1 hour.

The crosslinking reaction can be promoted by adding an initiator and/or a catalyst to the nonionic crosslinking agent to reduce the time and/or the temperature and/or the amount of nonionic crosslinking agent required to join the precursor particles together. Generally, however, the reaction is conducted in the absence of a catalyst.

The physical association of the precursor particles needs to be maintained during the reaction step so that sufficient interparticle crosslink bonds are formed. If forces or stresses sufficient to dissociate the precursor particles are present during the reaction step, the crosslink bonds between the precursor particles (interparticle crosslink bonds) may not be formed. The physical association of the precursor particles is typically maintained by insuring minimal dissociation forces or stresses are introduced during the reaction step.

Examples of various types of PAPC particles and the compositions of various liquid mixtures that can be used with the present invention is given below.

A. The Particulate Absorbent Polymeric Compositions or Particles Preferred for the Present Invention

The sheets of the present invention are formed from polymer materials capable of absorbing large quantities of liquids. (Such polymer materials are commonly referred to as "hydrogel", "hydrocolloid", or "superabsorbent" materials.) The sheets preferably comprise substantially water-insoluble, absorbent hydrogel-forming, polymer material. The specific polymer materials will be discussed herein with respect to those forming the PAPC particles.

Although the PAPC particles can have a size varying over a wide range, specific particle size distributions and sizes are preferred. For purposes of the present invention, particle size is defined for PAPC particles that do not have a large greatest dimension/smallest dimension ratio such as fibers (e.g., granules, flakes, or pulverulents) as the dimension of a PAPC particle which is determined by sieve size analysis. Thus, for example, a PAPC particle that is retained on a standard #30 sieve with 600 micron openings is considered to have a particle size greater than 600 microns, a PAPC particle that passes through the #30 sieve with 600 micron openings and is retained on a standard #35 sieve with 500 micron openings is considered to have a particle size between 500 and 600 microns, and a PAPC particle that passes through a #35 sieve with 500 micron openings is considered to have a particle size less than 500 microns. In preferred embodiments of the present invention, the PAPC particles will generally range in size from about 1 micron to about 2000 microns, more preferably from about 20 microns to about 1000 microns.

Further, for purposes of this invention, the mass average particle size of the PAPC particles is important in determining the characteristics and properties of the resultant sheets. The mass average particle size of a given sample of PAPC particles is defined as the particle size which is the average particle size of the sample on a mass basis. A method for determining the mass average particle size of a sample is described hereinafter in the Test Methods section. The mass average particle size of the PAPC particles will generally be from about 20 microns to about 1500 microns, more preferably from about 50 microns to about 1000 microns. In preferred embodiments of the present invention, the PAPC particles have a mass average particle size less than about 1000 microns, more preferably less than about 600 microns, most preferably less than about 500 microns. In especially preferred embodiments of the present invention, the mass average particle size of the PAPC particles is relatively small (i.e. the PAPC particles are fines). In these embodiments, the mass average particle size of the PAPC particles is less than about 300 microns, more preferably less than about 180 microns. In an exemplary embodiment, at least about 95% by weight of the PAPC particles have a particle size between about 150 microns and about 300 microns. In an alternative embodiment, at least about 95% by weight of the PAPC particles have a particle size between about 90 microns and about 180 microns. Narrow PAPC particle size distributions are preferred because they result in a higher porosity sheet due to the higher void fraction when densified versus broader PAPC particle size distributions with equivalent mass average particle sizes.

The particle size of materials having a large greatest dimension/smallest dimension such as fibers is typically defined by their largest dimension. For example, if absorbent, polymeric fibers (i.e. superabsorbent fibers) are used in the sheets of the present invention, the length of the fibers is used to define the "particle size." (The denier and/or the diameter of the fibers can also be specified.) In exemplary embodiments of the present invention, the fibers have a length greater than about 5 mm, preferably between about 10 mm and about 100 mm, more preferably between about 10 mm and about 50 mm.

The PAPC particles comprise substantially water-insoluble, absorbent hydrogel-forming, polymer material having a multiplicity of anionic, functional groups, such as sulfonic acid, and more typically carboxy, groups. Examples of polymer materials suitable for use as the PAPC particles herein include those which are prepared from polymerizable, unsaturated, acid-containing monomers. Thus, such monomers include the olefinically unsaturated acids and anhydrides which contain at least one carbon to carbon olefinic double bond. More specifically, these monomers can be selected from olefinically unsaturated carboxylic acids and acid anhydrides, olefinically unsaturated sulfonic acids, and mixtures thereof.

Some non-acid monomers can also be included, usually minor amounts, in preparing the PAPC particles herein. Such non-acid monomers can include, for example, the water-soluble or water-dispersible esters of the acid-containing monomers, as well as monomers which contain no carboxylic or sulfonic acid groups at all. Optional non-acid monomers can thus include monomers containing the following types of functional groups: carboxylic acid or sulfonic acid esters, hydroxyl groups, amide-groups, amino groups, nitrile groups and quaternary ammomium salt groups. These non-acid monomers are well-known materials and are described in greater detail, for example, in U.S. Patent 4,076,663 (Masuda et al), issued February 28, 1978, and in U.S. Patent 4,062,817 (Westerman), issued December 13, 1977.

Olefinically unsaturated carboxylic acid and carboxylic acid anhydride monomers include the acrylic acids typified by acrylic acid itself, methacrylic acid, ethacrylic acid, $\alpha$-chloroacrylic acid, $\alpha$-cyanoacrylic acid, $\beta$-methylacrylic acid (crotonic acid), $\alpha$-phenylacrylic acid, $\beta$-acryloxypropionic acid, sorbic acid, $\alpha$-chlorosorbic acid, angelic acid, cinnamic acid, p-chlorocinnamic acid, $\beta$-sterylacrylic acid, itaconic acid, citroconic acid, mesaconic acid, glutaconic acid, aconitic acid, maleic acid, fumaric acid, tricarboxyethylene and maleic acid anhydride.

Olefinically unsaturated sulfonic acid monomers include aliphatic or aromatic vinyl sulfonic acids such as vinylsulfonic acid, allyl sulfonic acid, vinyltoluene sulfonic acid and styrene sulfonic acid; acrylic and methacrylic sulfonic acid such as sulfoethyl acrylate, sulfoethyl methacrylate, sulfopropyl acrylate, sulfopropyl methacrylate, 2-hydroxy-3-methacryloxypropyl sulfonic acid and 2-acrylamide-2-methylpropane sulfonic acid.

Preferred polymer materials for use in the present invention contain carboxy groups. These polymers include hydrolyzed starch-acrylonitrile graft copolymers, partially neutralized starch-acrylonitrile graft copolymers, starch-acrylic acid graft copolymers, partially neutralized starch-acrylic acid graft copolymers, saponified vinyl acetate-acrylic ester copolymers, hydrolyzed acrylonitrile or acrylamide copolymers, slightly network crosslinked polymers of any of the foregoing copolymers, partially neutralized polyacrylic acid, and slightly network crosslinked polymers of partially neutralized polyacrylic acid. These polymers can be used either solely or in the form of a mixture of two or more different polymers. Examples of these polymer materials are disclosed in U.S. Patent 3,661,875, U.S. Patent 4,076,663, U.S. Patent 4,093,776, U.S. Patent 4,666,983, and U.S. Patent 4,734,478.

Most preferred polymer materials for use in making the PAPC particles are slightly network crosslinked polymers of partially neutralized polyacrylic acids and starch derivatives thereof. Most preferably, the PAPC particles comprise from about 50 to about 95%, preferably about 75%, neutralized, slightly network crosslinked, polyacrylic acid (i.e. poly (sodium acrylate/acrylic acid)).

As described above, the PAPC particles are preferably made from polymer materials that are slightly network crosslinked. Network crosslinking serves to render the polymer materials from which the PAPC particles are made substantially water-insoluble and, in part, determines the absorptive capacity and extractable polymer content characteristics of the PAPC particles and the resultant sheets. Processes for network crosslinking the polymers and typical network crosslinking agents are described in greater detail in the hereinbefore-referenced U.S. Patent 4,076,663.

The individual PAPC particles can be formed in any conventional manner. Typical and preferred processes for producing the individual PAPC particles are described in U.S. Patent Re. 32,649 (Brandt et al), issued April 19, 1988, U.S. Patent 4,666,983 (Tsubakimoto et al), issued May 19, 1987, and U.S. Patent 4,625,001 (Tsubakimoto et al), issued November 25, 1986.

In preferred embodiments of the present invention, the PAPC particles used to form the bonded particle aggregates are substantially dry. The term "substantially dry" is used herein to mean that the PAPC particles have a liquid content, typically water or other solution content, less than about 50%, preferably less than about 20%, more preferably less than about 10%, by weight of the PAPC particles. In general, the liquid content of the PAPC particles is in the range of from about 0.01% to about 5% by weight of the PAPC particles. The individual PAPC particles can be dried by any conventional method such as by heating. Alternatively, when the PAPC particles are formed using an aqueous reaction mixture, water can be removed from the reaction mixture by azeotropic distillation. The polymer-containing aqueous reaction mixture can also be treated with a dewatering solvent such as methanol. Combinations of these drying procedures can also be used. The dewatered mass of polymer material can then be chopped or pulverized to form substantially dry PAPC particles of substantially water-insoluble, absorbent, hydrogel-forming, polymer material.

Preferred PAPC particles of the present invention are those which exhibit a high absorptive capacity so that the resultant sheet formed from such PAPC particles also has a high absorptive capacity. Absorptive capacity refers to the capacity of a given polymer material to absorb liquids with which it comes into contact. Absorptive capacity can vary significantly with the nature of the liquid being absorbed and with the manner in which the liquid contacts the polymer material. For purposes of this invention, Absorptive Capacity is defined in terms of the amount of Synthetic Urine (as hereinafter defined) absorbed by any given polymer material in terms of grams of Synthetic Urine per gram of polymer material in a procedure hereinafter defined in the Test Methods section. Preferred PAPC particles of the present invention are those which have an Absorptive Capacity of at least about 20 grams, more preferably at least about 25 grams, of Synthetic Urine per gram of polymer material. Typically, the polymer materials of the PAPC particles herein have an Absorptive Capacity of from about 40 grams to about 70 grams of Synthetic Urine per gram of polymer material. Precursor particles having this relatively high absorptive capacity characteristic produce sheets that are especially useful in absorbent products, absorbent members, and absorbent articles since the resultant sheets formed from such PAPC particles can, by definition, hold desirably high amounts of discharged body exudates such as urine.

While all of the PAPC particles are preferably formed from the same polymer material with the same properties, this need not be the case. For example, some PAPC particles can comprise starch-acrylic acid graft copolymer while other PAPC particles can comprise a slightly network crosslinked polymer of partially neutralized polyacrylic acid. Further, the PAPC particles can vary in shape, absorptive capacity, or any other property or characteristic. In a preferred embodiment of the present invention, the PAPC particles consist essentially of slightly network crosslinked polymers of partially neutralized polyacrylic acid, each PAPC particle having similar properties.

B. Liquid Mixtures Containing Cationic Amino-Epichlorohydrin Adducts

Liquid mixtures using cationic amino-epichlorohydrin adducts as the cross-linking agent typically use an adduct of epichlorohydrin with certain types of monomeric or polymeric amines. These amino-epichlorohydrin adducts react with the polymer material of the absorbent PAPC particles, and in particular the anionic, typically carboxy, functional groups of these polymer materials to form a covalent, ester-type bond. In other words, the amino-epichlorohydrin adduct serves to crosslink the polymer material present in the absorbent PAPC particles. (The portions of the absorbent particle containing polymer material that has been effectively crosslinked with the amino-epichlorohydrin adduct swell less in the presence of aqueous body fluids relative to the other uncrosslinked portions of the particle.)

It is believed that these reacted amino-epichlorohydrin adducts primarily provide crosslinking at the surface of the absorbent PAPC particles. This is due to the fact that these adducts, and especially the polymeric resin versions of these adducts, are relatively large, cationic molecules. As a result, they are unable to penetrate inside the absorbent particles, and therefore can only react with polymer material at the surface thereof. In addition, the cationic functional groups (e.g., azetedinium groups) of these adducts, particularly polymeric resin versions, are believed to react very rapidly with the anionic, typically carboxy, functional groups of the polymer material of the absorbent particles, even at room temperature (i.e. at from about 18° to about 25°C). As a result, fairly modest levels (e.g., as low as about 1% by weight of the particles) of these amino-epichlorohydrin adducts are required to provide effective surface crosslinking of the polymer material present in the absorbent PAPC particles.

As used herein, "cationic amino-epichlorohydrin adduct" refers to the reaction product between epichlorohydrin and a monomeric or polymeric amine such that the resulting reaction product has at least two cationic functional groups. These adducts can be in the form of monomeric compounds (e.g., the reaction product of epichlorohydrin and ethylene diamine), or can be in polymeric form (e.g., the reaction product between epichlorohydrin, and polyamide-polyamines or polyethyleneimines). The polymeric versions of these cationic amino-epichlorohydrin adducts are typically referred to as "resins."

One type of amino compound which can be reacted with epichlorohydrin to form adducts useful in the present invention comprises monomeric di-, tri- and higher amines having primary or secondary amino groups in their structures. Examples of useful diamines of this type include bis-2-aminoethyl ether, N,N-dimethylethylenediamine, piperazine, and ethylenediamine. Examples of useful triamines of this type include N-aminoethyl piperazine, and dialkylene triamines such as diethylenetriamine, and dipropylenetriamine.

Such amine materials are reacted with epichlorohydrin to form the cationic amino-epichlorohydrin adducts useful as crosslinking agents herein. Preparation of these adducts, as well as a more complete description of the adducts themselves, can be found in U.S. Patent 4,310,593 (Gross), issued January 12, 1982, and in Ross et al, J. Organic Chemistry, Vol. 29, pp. 824-826 (1964).

In addition to monomeric amines, polymeric amines such as polyethyleneimines can also be used as the amino compound. A particularly desirable amino compound which can be reacted with epichlorohydrin to form preferred cationic polymeric adduct resins useful herein comprise certain polyamide-polyamines derived from polyalkylene polyamines and saturated $C_3$-$C_{10}$ dibasic carboxylic acids. Epichlorohydrin/polyamide-polyamine adducts of this kind are water-soluble, thermosetting cationic polymers which are well known in the art as wet strength resins for paper products.

In the preparation of polyamide-polyamines used to form this preferred class of cationic polymeric resins, a dicarboxylic acid is first reacted with a polyalkylene-polyamine, preferably in aqueous solution, under conditions such as to produce a water-soluble, long chain polyamide containing the recurring groups $-NH(C_nH_{2n}HN)_x-CORCO-$ where n and x are each 2 or more and R is the $C_1$ to $C_8$ alkylene group of the dicarboxylic acid.

A variety of polyalkylene polyamines including polyethylene polyamines, polypropylene polyamines, polybutylene polyamines and so on can be employed to prepare the polyamide-polyamine, of which the polyethylene polyamines represent an economically preferred class. More specifically, preferred polyalkylene polyamines used to prepare the cationic polymeric resins herein are polyamines containing two primary amine groups and at least one secondary amine group in which the nitrogen atoms are linked together by groups of the formula $-C_nH_{2n}-$ where n is a small integer greater than unity and the number of such groups in the molecule ranges from two up to about eight and preferably up to about four. The nitrogen atoms can be attached to adjacent carbon atoms in the group $-C_nH_{2n}-$ or to carbon atoms further apart, but not to the same carbon atom. Also contemplated is the use of such polyamines as diethylenetriamine, triethylenetetramine, tetraethylenepentamine, dipropylenetriamine, and the like, which can be obtained in reasonably pure form. Of all the foregoing, the most preferred are the polyethylene polyamines containing from two to four ethylene groups, two primary amine groups, and from one to three secondary amine groups.

Also contemplated for use herein are polyamines containing at least three amino groups with at least one of these groups being a tertiary amino group. Suitable polyamines of this type include methyl bis(3-aminopropyl)amine, methyl bis(2-aminoethyl)amine, N-(2-aminoethyl)piperazine, 4,7-dimethyltriethylenetetramine and the like.

The dicarboxylic acids which can be reacted with the foregoing polyamines to form the polyamide-polyamines of the preferred cationic polymeric resins useful herein comprise the saturated aliphatic $C_3$-$C_{10}$ dicarboxylic acids. More preferred are those containing from 3 to 8 carbon atoms, such as malonic, succinic, glutaric, adipic, and so on, together with diglycolic acid. Of these, diglycolic acid and the saturated aliphatic dicarboxylic acids having from 4 to 6 carbon atoms in the molecule, namely, succinic, glutaric and adipic are most preferred. Blends of two or more of these dicarboxylic acids can also be used, as well as blends of one or more of these with higher saturated aliphatic dicarboxylic acids such as azelaic and sebacic, as long as the resulting long chain polyamide-polyamine is water-soluble or at least water-dispersible.

The polyamide-polyamine materials prepared from the foregoing polyamines and dicarboxylic acids are reacted with epichlorohydrin to form the cationic polymeric amino-epichlorohydrin resins preferred for use herein as the crosslinking agent. Preparation of such materials is describe in greater detail in U.S. Patent 2,926,116 (Keim), issued February 23, 1960, U.S. Patent 2,926,154 (Keim), issued February 23, 1960, and U.S. Patent 3,332,901 (Keim), issued July 25, 1967.

The cationic polyamide-polyamine-epichlorohydrin resins preferred for use herein as crosslinking agents are commercially marketed by Hercules Inc. under the trade name Kymene®. Especially useful are Kymene® 557H, Kymene® 557LX and Kymene® Plus, which are the epichlorohydrin adducts of polyamide-polyamines which are the reaction products of diethylenetriamine and adipic acid. They are typically marketed in the form of aqueous solutions of the cationic resin material containing from about 10% to about 33% by weight of the resin active.

Other components or agents can be used as aids in preparing the liquid mixture. For example, water is typically used with the adduct to form an aqueous liquid mixture thereof. Water promotes the uniform dispersion of the adduct on the surface of the PAPC particles and causes permeation of the adduct into the surface regions of these particles. Water also promotes a stronger physical association between the treated PAPC particles, providing greater integrity of the resultant interparticle bonded crosslinked aggregates. In the present invention, water is used in an amount of less than about 25 parts by weight (i.e. from 0 to about 25 parts by weight), preferably in the range of from about 3 to about 15 parts by weight, more preferably in the range of from about 5 to about 10 parts by weight, per 100 parts by weight of the PAPC particles. The actual amount of water used can vary depending upon the type of adduct used, the type of polymer material used in forming the PAPC particles, the particle size of these PAPC particles, the inclusion of other optional components (e.g., glycerol) and like factors.

Although not absolutely necessary, organic solvents can be used, usually to promote uniform dispersion of the cationic amino-epichlorohydrin adduct onto the surface of the PAPC particles. These organic solvents are typically hydrophilic, and can include lower alcohols such as methanol and ethanol; amides such as N,N-dimethylformamide and N,N-diethylformamide; and sulfoxides such as dimethylsulfoxide. If a hydrophilic solvent is used, it is in an amount of less than about 20 parts by weight (i.e. from 0 to about 20 parts by weight), preferably in the range of from about 5 to about 15 parts by weight, more preferably in the range of from about 8 to about 12 parts by weight, per 100 parts by weight of the PAPC particles. The actual amount of hydrophilic solvent used can vary depending upon the adduct used, the polymer material used forming the PAPC particles, the particle size of these PAPC particles and like factors.

As previously noted, the use of hydrophilic organic solvents is not necessarily required in preparing bonded particle aggregates when cationic amino-epichlorohydrin adducts are the cross-linking agent. Indeed, it can be desirable to avoid the use of such organic solvents. Such solvents typically need to be removed from the aggregate before it is suitable for its intended use. The removal of organic solvents is frequently an energy and process intensive, and adds additional processing costs. In particular, some hydrophilic solvents, such as isoproponal or t-butanol, can cause the amino-epichlorohydrin adduct to precipitate out of solution and are therefore undesirable for this reason. Indeed, the only solvents typically used in preparing bonded particle aggregates when the cross-linking agent is a cationic amino-epichlorohydrin adduct are the lower alcohols such as methanol and ethanol that are not too energy or process intensive to remove, and do not cause the amino-epichlorohydrin adduct to precipitate out of aqueous solution.

Other optional components can also be used with the cationic amino-epichlorohydrin adduct, and especially aqueous liquid mixtures thereof. It is particularly preferred that the liquid mixture comprising the cationic amino-epichlorohydrin adduct include a plasticizer, especially when the treated PAPC particles are ambient temperature cured as described hereafter. In the absence of a plasticizer, the treated PAPC particles, when formed into the interparticle bonded aggregates, can be relatively brittle, and thus more difficult to handle, especially in making the ultimately desired absorbent structures. Inclusion of a plasticizer in the liquid mixture insures that the resulting interparticle bonded aggregates (when ambient temperature cured) form relatively flexible porous, absorbent sheets, particularly flexible, porous, absorbent sheets of the interparticle bonded aggregates. These flexible, stretchable sheets are relatively easy to handle in making the ultimately desired absorbent structures.

Suitable plasticizers include water, alone or in combination with other components such as glycerol, propylene glycol (i.e. 1,2-propanediol), 1,3-propanediol, or ethylene glycol, sorbitol, sucrose, polymeric solutions such as those involving polyvinyl alcohol, ester PAPCs of polyvinyl alcohol, or polyethylene glycol, or mixtures thereof. These other components in the plasticizer, such as glycerol, are believed to act as humectants, coplasticizers or both, with water being the primary plasticizer. The preferred plasticizer for use with liquid mixtures of the cationic amino-epichlorohydrin adduct is a mixture of glycerol and water in a weight ratio of from about 0.5:1 to about 2:1, preferably from about 0.8:1 to about 1.7:1.

The actual amount of plasticizer used can vary depending upon the particular plasticizer used, the type of polymer material used in forming the PAPC particles, and the particular flexibility effects desired from the plasticizer. Typically, the plasticizer is used in an amount of from about 5 to about 100 parts by weight, preferably from about 5 to about 60 parts by weight, more preferably from about 10 to about 30 parts by weight, most preferably from about 15 to about 20 parts by weight, per 100 parts by weight of the PAPC particles.

C. Liquid Mixtures Containing Alternative Cross-Linking Agents

Alternative but less preferable crosslinking agents useful in the present invention include, for example, compounds having at least two polymerizable double bonds; compounds having at least one polymerizable double bond and at least one functional group reactive with the polymer material; compounds other than cationic amino-epichlorohydrin adducts having at least two functional groups reactive with the polymer material; polyvalent metal compounds; or monomers as described herein. Specific alternative crosslinking agents useful in the present invention are described in the hereinbefore referenced U.S. Patent 4,076,663 and U.S. Patent Re. 32,649 which are incorporated herein by reference.

Where carboxy groups are present on or in the polymer materials (i.e., the polymer chains) of the PAPC particles, preferred alternative crosslinking agents are typically nonionic and possess at least two functional groups per molecule capable of reacting with the carboxy group. Preferred alternative crosslinking agents include polyhydric alcohols such as ethylene glycol, diethylene glycol, triethylene glycol, tetraethylene glycol, polyethylene glycol, glycerol (1,2,3-propanetriol), polyglycerol, propylene glycol, 1, 2-propanediol, 1, 3-propanediol, trimethylol propane, diethanolamine, triethanolamine, polyoxypropylene oxyethylene-oxypropyle block copolymer, sorbitan fatty acid esters, polyexyethylene sorbitan fatty acid esters, pentaerythritol, and sorbitol; polyglycidyl ether compounds such as ethylene glycol diglycidyl ether, polyethylene glycol diglycidyl ether, glycerol polyglycidyl ether, diglycerol polyglycidyl ether, polyglycerol polyglycidyl ether, sorbitol polyglycidyl ether, pentaerythritol polyglycidyl ether, propylene glycol diglycidyl ether, and propylene glycol diglycidyl ether; polyaziridine compounds such as 2, 2-bishydroxymethyl butanol-tris[3-(i-aziridine) propionate], 1, 6-hexamethyl tolulene diethylene urea, and diphenyl methane-bis-4, 4'-N,N'-diethylene urea; haloepoxy compounds such as epichlorohydrin and $\alpha$-methylfluorohydrin; polyaldehyde compounds such as glutaraldehyde and glyoxazole; polyamine compounds such as ethylene diamine, diethylene triamine, triethylene tetramine, tetraethylene pentamine, pentaethylene hexamine, and polyethylene imine; and polyisocyanate compounds such as 2, 4-tolulene diisocyanate and hexamethylene diisocyanate.

One crosslinking agent or two or more substantially mutually unreactive crosslinking agents selected from the group mentioned above may be used. Particularly preferred alternative crosslinking agents for use herein with carboxy-containing polymer chains are ethylene glycol; glycerol; trimethylol propane; 1, 2-propanediol; and 1, 3-propanediol.

The proportion of the alternative crosslinking agent to be used in the present invention is in the range of from about 0.01 parts to about 30 parts by weight, preferably from about 0.5 parts to about 10 parts by weight, most preferably from about 1 part to about 5 parts by weight, per 100 parts by weight of the PAPC particles.

In the present invention, other materials or agents can be used with the alternative crosslinking agent(s) as an aid in producing crosslinked aggregates or in promoting or assisting in the reaction of the crosslinking agent with the polymer material of the PAPC particles.

For example, water may be used in conjunction with the alternative crosslinking agent. The water functions to promote uniform dispersion of the crosslinking agent on the surface of the PAPC particles and permeation of the crosslinking agent into the surface region of the PAPC particles. The water also promotes stronger physical association between the PAPC particles of the prereacted aggregates, and the dry and swollen integrity of the resultant interparticle crosslinked aggregates. The water is used in a proportion of less than about 20 parts by weight (0 parts to about 20 parts by weight), preferably in the range of from about 0.01 parts to about 20 parts by weight, more preferably in the range of from about 0.1 parts to about 10 parts by weight, based on 100 parts by weight of the PAPC particles. The actual amount of water to be used will vary depending upon the kind of polymer material and the particle size of the PAPC particles.

Organic solvents may also be used in conjunction with the alternative crosslinking agent. The organic solvents are used to promote uniform dispersion of the crosslinking agent on the surface of the PAPC particles. The organic solvents are preferably hydrophilic organic solvents. Useful hydrophilic organic solvents include lower alcohols such as methanol, ethanol, n-propanol, isopropanol, n-butanol, isobutanol, sec-butanol and t-butanol; ketones such as acetone, methylethyl ketone, and methylisobutyl ketone; ethers such as dioxane, tetrahydrofuran, and diethyl ether; amides such as N,N-dimethylformamide and N,N-diethylformamide; and sulfoxides such as dimethyl sulfoxide. The hydrophilic organic solvent is used in a proportion of less than about 60 parts by weight (0 parts to about 60 parts by weight), preferably in the range of from about 0.01 parts to about 60 parts by weight, more preferably from about 1 part to about 20 parts by weight, based on 100 parts by weight of the PAPC particles. The actual amount of hydrophilic organic solvent to be used will vary depending upon the kind of polymer material and the particle size of the PAPC particles.

The alternative crosslinking agent may also be used in a mixture with water and one or more hydrophilic organic solvents. It has been found that the use of a water/crosslinking agent solution provides the greatest penetration of the crosslinker into the surface region of the PAPC particles while a solution of hydrophilic organic solvent/crosslinking agent provides minimal penetration of the crosslinker. However, a mixture of all three agents is preferred in order to control the amount of the penetration of the crosslinking agent into the surface region of the PAPC particles. Specifically, it has been found that the higher the water to organic solvent component ratio, the deeper the crosslinker penetration, the greater the fluid stability of the aggregates under stress, and the greater the reduction in the resultant absorptive capacity of the crosslinked aggregates. Typically, the ratio of water to hydrophilic organic solvent in the solution will be in the range of from about 10:1 to about 1:10. The hydrophilic organic solvent/water/interparticle crosslinking agent solution is used in a proportion less than about 60 parts by weight (0 parts to about 60 parts by weight), preferably in the range of from about 0.01 parts to about 60 parts by weight, more preferably from about 1 part to about 20 parts by weight, based on 100 parts by weight of the PAPC particles.

Other optional components may also be mixed with the solution containing the alternative crosslinking agent. For example, an initiator, a catalyst, or non-acid co-monomer materials may be added. Examples of these materials suitable for use herein are described in the hereinbefore referenced U.S. Patent Re. 32,649.

EXEMPLARY EMBODIMENTS OF THE PRESENT INVENTION

PAPC sheets having a densities around 1 gram/cubic centimeters and a thickness around .05 inches have proven to be useful for a number of applications including for use in absorbent cores of diapers. Examples of how to make such sheets are described below.

EXAMPLE 1

The PAPC particles desired to be used have a size between 150-300 microns. For PAPC particles of this size it is preferred to layer between 0.1-0.3 grams of PAPC per square inch of the support means or conveyor.

The composition of the liquid mixture to be used is given below, amounts are expressed in parts per 100 parts PAPC. The liquid mixture comprises:

5.0 parts Kymene® Plus (30% resin active)

12.7 parts glycerol

7.1 parts water

Therefore it is preferred that the ratio of parts of liquid mixture per 100 parts of PAPC be 24.8. The amount of liquid mixture (LM) that is preferred to be sprayed per square inch of conveyor can be calculated from the ratio of the parts of liquid mixture per 100 parts of PAPC by:

$$\frac{\text{grams of LM}}{\text{square inch per layer}} = \frac{\text{grams of PAPC}}{\text{square inch per layer}} * \frac{\text{grams LM}}{\text{grams PAPC}}$$

Any initial spraying step that is to be performed and the first spraying step thereafter would use half the amount calculated above. For this Example if we are to layer 0.2 grams of PAPC per layer per square inch, it is preferred to spray approximately 0.025 grams of liquid mixture per square inch of support means for the initial spraying step and the first spraying step thereafter, and to spray approximately 0.05 grams per square inch for all the other spraying steps.

The layering of the PAPC is desired to be accomplished by the vibrating feeder from Solids Flow Control described above.

The spraying of the liquid mixture is done by a model 6218-1/4 JAU from Spraying Systems Co., described above. For the first two applications of liquid mixture it is desired that the sprayers deliver the liquid mixture to the conveyor at a rate of 39.8 grams/min. For the subsequent spraying steps it is desired that the sprayer deliver the liquid mixture to the conveyor at a rate of 79.6 grams/min.

The conveyor desired to be used is a moving conveyor made from polyurethane, and travels at a speed of 27 ft/min.

The desired pressure applicators are a pair of compaction rolls having 8 inch diameters and being 12 inches wide. The top and bottom rolls are coated with the 934 plasma coating described above.

The Ambient temperature will be 72°F/22.2°C.

STEP 1:   To begin one would initially spray a predetermined area of the conveyor with the liquid mixture in an amount substantially equal to 0.025 grams of liquid mixture per square inch of conveyor.

STEP 2:   Following this one would layer substantially continuously 0.2 grams of the PAPC particles per square inch of conveyor onto the same predetermined area of the conveyor.

STEP 3:   The layered PAPC on the predetermined area of the conveyor is then sprayed with liquid mixture in an amount substantially equal to 0.025 grams of liquid mixture per square inch of conveyor.

STEP 4:   Following this one would layer substantially continuously 0.2 grams of the PAPC particles per square inch of conveyor onto the same predetermined area of the conveyor.

STEP 5:   The layered PAPC on the predetermined area of the conveyor is then sprayed with liquid mixture in an amount substantially equal to 0.050 grams of liquid mixture per square inch of conveyor.

STEP 6:   Steps 4 and 5 are then repeated, in order, 3 more times, giving a total of one initial spraying step and five post-layering spraying steps for a total of 0.25 grams of liquid mixture per square inch of conveyor and a total of five layering steps for a total of 1 gram of PAPC per square inch of conveyor.

STEP 7:   The layered PAPC is then passed through the compaction rolls. It is desired to have a gap between the compaction rolls of .035 inches. This produces a sheet of PAPC having a density of 0.995 grams per cubic centimeters.

STEP 8: The sheet of PAPC is then cured by placing it in a plastic bag and allowing it to sit at the ambient temperature for 48 hours.

The above method would result in an absorbent sheet of PAPC having good flexibility, gel blocking and wet integrity properties.

EXAMPLE 2

All of the conditions and equipment to be used for this Example will be the same as Example 1 except that a different liquid mixture will be used. In this Example the liquid mixture would contain a non-ionic cross-linking agent, i.e. glycerol. The composition of the liquid mixture is given below, the amounts are expressed in parts per 100 parts of PAPC:
5.0 parts of methanol
4.0 parts of glycerol
8.8 parts of water
As stated above all other equipment, materials and conditions would be the same as in the previous example including using PAPC particles between 150-300 microns.

STEP 1: To begin one would initially spray a predetermined area of the conveyor with the liquid mixture in an amount substantially equal to 0.018 grams of liquid mixture per square inch of conveyor.

STEP 2: Following this one would layer substantially continuously 0.2 grams of the PAPC particles per square inch of conveyor onto the same predetermined area of the conveyor.

STEP 3: The layered PAPC on the predetermined area of the conveyor is then sprayed with liquid mixture in an amount substantially equal to 0.018 grams of liquid mixture per square inch of conveyor.

STEP 4: Following this one would layer substantially continuously 0.2 grams of the PAPC particles per square inch of conveyor onto the same predetermined area of the conveyor.

STEP 5: The layered PAPC on the predetermined area of the conveyor is then sprayed with liquid mixture in an amount substantially equal to 0.036 grams of liquid mixture per square inch of conveyor.

STEP 6: Steps 4 and 5 are then repeated, in order, 3 more times, giving a total of one initial spraying step and five post-layering spraying steps for a total of 0.18 grams of liquid mixture per square inch of conveyor and a total of five layering steps for a total of 1 gram of PAPC per square inch of conveyor.

STEP 7: The layered PAPC is then passed through the compaction rolls. It is desired to have a gap between the compaction rolls of .035 inches. This produces a sheet of PAPC having a density of 1.04 grams per cubic centimeters.

STEP 8: The sheet of PAPC will then be cured by heating the sheet to about 190°C for about 45 minutes.

## Claims

1. A method for making a sheet from particulate substantially water-insoluble, absorbent hydrogel-forming polymer materials, said method characterized by:

    (a) layering a predetermined amount of said particulate substantially water-insoluble, absorbent hydrogel-forming polymer material so as to substantially cover a predetermined area of a support means;
    (b) spraying said layered particulate material in said predetermined area with an amount of a liquid mixture comprising water and a cross-linking agent for the polymers in said particulate material, said amount being sufficient to cause effective surface cross-linking of said particulate material when cured; and
    (c) applying pressure to said layered particulate material by passing said layer through a pair of opposing pressure applicators, thereby forming a cohesive sheet.

2. The method according to Claim 1 further including the step of curing said sheet by allowing said sheet to sit for a predetermined amount of time at a predetermined temperature, thereby providing for effective surface cross-linking of said particulate material such that said sheet will absorb liquid upon contact and remain in sheet form without separating into individual particles.

3. The method according to any of the preceding claims comprising the step of initially spraying said predetermined area of said surface with a predetermined amount of liquid mixture before layering said predetermined amount of said particulate material onto said conveyor.

4. The method according to any of the preceding claims wherein the steps recited in (a) and (b) of Claim 1 are repeated, in order, at least once before applying pressure to said layered particulate material.

5. The method according to any of the preceding claims wherein the outermost edges of said layered particulate material are trimmed off prior to compaction.

6. The method according to any of the preceding claims wherein said liquid mixture comprises a cationic amino-epichlorohydrin adduct, glycerol and water.

7. The method of Claim 6 wherein said curing step comprises storing said sheet at a temperature range from about 18° to about 35°C from about 12 to about 48 hours.

8. The method according to Claims 1-5 wherein said liquid mixture comprises glycerol and water.

9. The method according to Claim 8 wherein said liquid. mixture further comprises methanol.

10. The method according to Claim 8 or 9 wherein said curing step comprises heating said sheet to a temperature in the range from about 180° to about 200°C for from about 30 minutes to about 1 hour.

**Patentansprüche**

1. Ein Verfahren zur Herstellung eines Blattes aus teilchenförmigen, im wesentlichen wasserunlöslichen, absorbierenden Hydrogel-bildenden Polymermaterialien, wobei das genannte Verfahren gekennzeichnet ist durch:

    (a) schichtweises Ablegen einer vorherbestimmten Menge des genannten teilchenförmigen, im wesentlichen wasserunlöslichen, absorbierenden Hydrogel-bildenden Polymermaterials, um einen vorherbestimmten Bereich eines Trägermittels im wesentlichen zu bedecken;
    (b) Besprühen des genannten schichtweise abgelegten teilchenförmigen Materials in dem genannten vorherbestimmten Bereich mit einer Menge einer flüssigen Mischung, die Wasser und ein Vernetzungsmittel für die Polymeren in dem genannten teilchenförmigen Material umfaßt, wobei die genannte Menge ausreicht, um beim Härten eine wirksame Oberflächenvernetzung des genannten teilchenförmigen Materials zu bewirken; und
    (c) Aufbringen von Druck auf das genannte schichtweise abgelegte teilchenförmige Material durch Hindurchführen der genannten Schichte durch ein Paar einander gegenüberliegender Druckapplikatoren, wodurch ein kohäsives Blatt gebildet wird.

2. Das Verfahren nach Anspruch 1, das weiters den Schritt der Härtung des genannten Blattes umfaßt, indem das genannte Blatt während eines vorherbestimmten Zeitraums bei einer vorherbestimmten Temperatur verweilen gelassen wird, wodurch für eine wirksame Oberflächenvernetzung des genannten teilchenförmigen Materials gesorgt wird, sodaß das genannte Blatt beim Kontakt mit Flüssigkeit diese absorbieren und in Blattform bleiben wird, ohne daß es sich in einzelne Teilchen auftrennt.

3. Das Verfahren nach einem der vorhergehenden Ansprüche, welches den Schritt des anfänglichen Besprühens des genannten vorherbestimmten Bereichs der genannten Oberfläche mit einer vorherbestimmten Menge flüssiger Mischung vor dem schichtweisen Ablegen der vorherbestimmten Menge des genannten teilchenförmigen Materials auf das genannte Fördermittel umfaßt.

4. Das Verfahren nach einem der vorhergehenden Ansprüche, in welchem die in (a) und (b) des Anspruchs 1 angegebenen Schritte der Reihe nach mindestens einmal wiederholt werden, bevor auf das genannte schichtweise abgelegte teilchenförmige Material Druck aufgebracht wird.

5. Das Verfahren nach einem der vorhergehenden Ansprüche, in welchem die äußersten Ränder des genannten schichtweise abgelegten Materials vor der Kompaktierung beschnitten werden.

**6.** Das Verfahren nach einem der vorhergehenden Ansprüche, in welchem die genannte flüssige Mischung ein kationisches Amino-Epichlorhydrin-Addukt, Glycerin und Wasser umfaßt.

**7.** Das Verfahren nach Anspruch 6, in welchem der genannte Härtungsschritt ein Lagern des genannten Blattes in einem Temperaturbereich von etwa 18°C bis etwa 35°C während etwa 12 bis etwa 48 Stunden umfaßt.

**8.** Das Verfahren nach den Ansprüchen 1 bis 5, in welchem die genannte flüssige Mischung Glycerin und Wasser umfaßt.

**9.** Das Verfahren nach Anspruch 8, in welchem die genannte flüssige Mischung außerdem Methanol umfaßt.

**10.** Das Verfahren nach Anspruch 8 oder 9, in welchem der genannte Härtungsschritt das Erhitzen des genannten Blattes auf eine Temperatur im Bereich von etwa 180°C bis etwa 200°C während etwa 30 Minuten bis etwa 1 Stunde umfaßt.

## Revendications

**1.** Procédé de fabrication d'une feuille à partir de matières polymères particulaires formatrices d'hydrogels, absorbantes, sensiblement insolubles dans l'eau, ledit procédé étant caractérisé par les étapes suivantes :

(a) on applique en couche une quantité prédéterminée de ladite matière polymère particulaire formatrice d'hydrogel, absorbante, sensiblement insoluble dans l'eau de manière à recouvrir sensiblement une zone prédéterminée d'un moyen de support,
(b) on pulvérise ladite matière particulaire appliquée en couche dans ladite zone prédéterminée avec une quantité d'un mélange liquide comprenant de l'eau et un agent de réticulation pour les polymères dans ladite matière particulaire, ladite quantité étant suffisante pour provoquer une réticulation superficielle efficace de ladite matière particulaire lorsqu'elle durcit, et
(b) on applique une certaine pression à ladite matière particulaire appliquée en couche en faisant passer ladite couche à travers deux applicateurs de pression opposés, de façon à former une couche cohésive.

**2.** Procédé selon la revendication 1, comprenant, en outre, l'étape de durcissement de ladite feuille en permettant à ladite feuille de rester à une température prédéterminée pendant une période de temps prédéterminée, de façon à effectuer une réticulation superficielle efficace de ladite matière particulaire telle que ladite feuille absorbe les liquides par contact et reste sous forme de feuille sans se séparer en particules individuelles.

**3.** Procédé selon l'une quelconque des revendications précédentes, comprenant l'étape de pulvérisation initiale de ladite zone prédéterminée de ladite surface avec une quantité prédéterminée de mélange liquide avant d'appliquer en couche ladite quantité prédéterminée de ladite matière particulaire sur ledit moyen de support.

**4.** Procédé selon l'une quelconque des revendications précédentes, dans lequel les étapes mentionnées dans (a) et (b) de la revendication 1 sont répétées, dans l'ordre, au moins une fois avant l'application de pression à ladite matière particulaire appliquée en couche.

**5.** Procédé selon l'une quelconque des revendications précédentes, dans lequel les bords externes de ladite matière particulaire appliquée en couche sont rognés avant compactage.

**6.** Procédé selon l'une quelconque des revendications précédentes, dans lequel ledit mélange liquide comprend un produit d'addition cationique d'amino-épichlorhydrine, du glycérol et de l'eau.

**7.** Procédé selon la revendication 6, dans lequel ladite étape de durcissement comprend le stockage de ladite feuille dans une plage de températures d'environ 18 à environ 35°C pendant environ 12 à environ 48 heures.

**8.** Procédé selon l'une quelconque des revendications 1 à 5, dans lequel ledit mélange liquide comprend du glycérol et de l'eau.

**9.** Procédé selon la revendication 8, dans lequel ledit mélange liquide comprend, en outre, du méthanol.

10. Procédé selon la revendication 8 ou 9, dans lequel ladite étape de durcissement comprend le chauffage de ladite feuille à une température dans la plage d'environ 180 à environ 200°C pendant environ 30 minutes à environ 1 heure.

Fig. 1